# EUROPEAN PATENT SPECIFICATION

(11) **EP 2 576 599 B1**
(45) Date of publication and mention of the grant of the patent: **18.03.2015**
(21) Application number: 11716841.9
(22) Date of filing: 29.04.2011
(51) Int. Cl.: C07K 14/195, C12Q 1/68

(54) **A GENETICALLY MODIFIED BACTERIUM OF THE SPECIES LISTERIA MONOCYTOGENES**
GENETISCH MODIFIZIERTES BAKTERIUM DER SPEZIES LISTERIA MONOCYTOGENES
BACTÉRIE GÉNÉTIQUEMENT MODIFIÉE DE L'ESPÈCE LISTERIA MONOCYTOGENES

(30) Priority: 02.06.2010 EP 10005731
(43) Date of publication of application: 10.04.2013
(73) Proprietor: Merck Patent GmbH, 64293 Darmstadt (DE)
(72) Inventor: ROSSMANITH, Peter, A-2531 Gaaden (AT); FRUEHWIRTH, Karin, A-2870 Aspang (AT); WAGNER, Martin, A-1180 Wien (AT); FUCHS, Sabine, A-3602 Kirchstetten (AT)
(86) International application number: PCT/EP2011/002147
(87) International publication number: WO 2011/150999

(56) References cited:
- WO-A1-2008/017097
- ROSSMANITH PETER ET AL: "Proof of Concept for Recombinant Cellular Controls in Quantitative Molecular Pathogen Detection", APPLIED AND ENVIRONMENTAL MICROBIOLOGY, AMERICAN SOCIETY FOR MICROBIOLOGY, US, vol. 77, no. 7, 11 February 2011 (2011-02-11), pages 2531-2533, XP009151282, ISSN: 0099-2240
- P. ROSSMANITH ET AL: "The challenge to quantify Listeria monocytogenes- a model leading to new aspects in molecular biological food pathogen detection", JOURNAL OF APPLIED MICROBIOLOGY, vol. 110, no. 3, 1 March 2011 (2011-03-01) , pages 605-617, XP55004894, ISSN: 1364-5072, DOI: 10.1111/j.1365-2672.2010.04915.x
- ROSSMANITH P ET AL: "Detection of Listeria monocytogenes in food using a combined enrichment/real-time PCR method targeting the prfA gene", RESEARCH IN MICROBIOLOGY, ELSEVIER, AMSTERDAM, NL, vol. 157, no. 8, 1 October 2006 (2006-10-01), pages 763-771, XP025101101, ISSN: 0923-2508, DOI: 10.1016/J.RESMIC.2006.03.003 [retrieved on 2006-10-01] cited in the application
- MURPHY ET AL: "Construction and evaluation of a microbiological positive process internal control for PCR-based examination of food samples for Listeria monocytogenes and Salmonella enterica", INTERNATIONAL JOURNAL OF FOOD MICROBIOLOGY, ELSEVIER SCIENCE PUBLISHERS, AMSTERDAM, NL, vol. 120, no. 1-2, 30 November 2007 (2007-11-30), pages 110-119, XP022373142, ISSN: 0168-1605, DOI: 10.1016/J.IJFOODMICRO.2007.06.006 cited in the application
- O'GRADY J ET AL: "Rapid detection of Listeria monocytogenes in food using culture enrichment combined with real-time PCR", FOOD MICROBIOLOGY, ACADEMIC PRESS LTD, LONDON, GB, vol. 26, no. 1, 1 February 2009 (2009-02-01), pages 4-7, XP025686774, ISSN: 0740-0020, DOI: 10.1016/J.FM.2008.08.009 [retrieved on 2008-09-13]
- MATTISON K ET AL: "The feline calicivirus as a sample process control for the detection of food and waterborne RNA viruses", INTERNATIONAL JOURNAL OF FOOD MICROBIOLOGY, ELSEVIER SCIENCE PUBLISHERS, AMSTERDAM, NL, vol. 132, no. 1, 1 June 2009 (2009-06-01), pages 73-77, XP026094588, ISSN: 0168-1605, DOI: 10.1016/J.IJFOODMICRO.2009.04.002 [retrieved on 2009-04-09] cited in the application
- HOORFAR ET AL: "Practical considerations in design of internal amplification controls for diagnostic PCR assays.", JOURNAL OF CLINICAL MICROBIOLOGY, vol. 42, no. 5, 1 May 2004 (2004-05-01), pages 1863-1868, XP55004901, ISSN: 0095-1137 cited in the application
- BOECKMANN REGINE ET AL: "Specific binding of the Listeria monocytogenes transcriptional regulator PrfA to target sequences requires additional factor(s) and is influenced by iron", MOLECULAR MICROBIOLOGY, vol. 22, no. 4, 1996, pages 643-653, XP000002656891, ISSN: 0950-382X cited in the application

### SEQUENCE LISTING

<110> Merck Patent GmbH
<120> A non-natural mutant of the bacterial species Listeria monocytogenes
<130> I10/116
<160> 7
<170> PatentIn version 3.5
<210> 1
   <211> 100
   <212> DNA
   <213> Artificial Sequence
<220>
   <223> Internal Amplification Control sequence (IAC)
<300>
   <301> Rossmanith et al. <302> Detection of Listeria monocytogenesin food using a combined enrichement/real-time PCR method targeting the prfA gene <303> Res. Microbiol. <304> 157 <306> 763-771 <307> 2006-04-03
<400> 1
   gatacagaaa catcggttgg cgtattcgaa atgtccgttc ggttggcgct atgaagagat      60
   acgcggtgga acctggaacc tgatggcatc aagattacac                           100
<210> 2
   <211> 21
   <212> DNA
   <213> Artificial Sequence
<220>
   <223> Primer Lip1
<300>
   <301> D'Agostino et al. <302> A validated PCR-based method to detect Listeria monocytogenes using raw milf as a food model towards an international standard <303> J. Food. Prot. <304> 67 <306> 1646-1655 <307> 2004
<400> 2
   gatacagaaa catcggttgg c 21
<210> 3
   <211> 22
   <212> DNA
   <213> Artificial Sequence
<220>
   <223> Primer Lip2
<300>
   <301> D'Agostino et al. <302> A validated PCR-based method to detect Listeria monocytogenes using raw milf as a food model towards an international standard <303> J. Food Prot. <304> 67 <306> 1646-1655 <307> 2004
<400> 3
   gtgtaatctt gatgccatca gg 22
<210> 4
   <211> 23
   <212> DNA
   <213> Artificial Sequence
<220>
   <223> pLucLm4: Probe binding IAC
<300>
   <301> Rossmanith et al. <302> Detection of Listeria monocytogenes in food using a combined enrichment/real-time PCR method targeting the prfA gene <303> Res. Microbiol. <304> 157 <306> 763-771 <307> 2006
<400> 4
   ttcgaaatgt ccgttcggtt ggc 23
<210> 5
   <211> 21
   <212> DNA
   <213> Artificial Sequence
<220>
   <223> LipProbe: Probe binding prfA locus
<300>
   <301> Rossmanith et al. <302> Detection of Listeria monocytogenes in food using a combined enrichment/real-time PCR method targeting the prfA gene <303> Res. Microbiol. <304> 157 <306> 763-771 <307> 2006
<400> 5
   caggattaaa agttgaccgc a 21
<210> 6
   <211> 31
   <212> DNA
   <213> Artificial Sequence
<220>
   <223> LipBam Primer
<400> 6
   gcgcggatcc gatacagaaa catcggttgg c 31
<210> 7
   <211> 32
   <212> DNA
   <213> Artificial Sequence
<220>
   <223> LipSal Primer
<400> 7
   gcgcgtcgac gtgtaatctt gatgccatca gg 32

## Description

The present invention relates to a genetically modified bacterium of the species *Listeria monocytogenes,* wherein the genomic locus of the transcriptional factor PrfA has been deleted, characterised in that it comprises on genomic level an artificial sequence that acts as internal amplification control, the use of this bacterium as well as a method for detecting and determining qualitatively and /or quantitatively the occurrence of wild type *Listeria monocytogenes* in a sample suspected to be contaminated with said micro-organism and a kit.

### Background of the invention:

*Listeria monocytogenes* is a pathogenic gram-positive bacterium. Causing Listeriosis it is one of the most virulent food borne pathogens. The detection of pathogens in samples like food samples or clinical samples like blood, tissue or feces is becoming increasingly important. However, in order to clearly identify and optionally to quantify the cells comprised in a sample reliable methods for their detection and quantification have to be provided.

Since the first implementation of polymerase chain reaction (PCR) this molecular biological tool has been developed to a method providing many opportunities for improved detection of micro-organisms. An important prerequisite for PCR based methods to become internationally recognized standard is the internal amplification control (IAC). An IAC is a non-target DNA sequence present in the same sample tube, which is co-amplified simultaneously with the target sequence. In a PCR without an IAC, a negative result is not definite as it can mean that there was no target sequence present but also that the amplification reaction was inhibited by various factors. Therefore the European Standardization Committee in collaboration with the International Standard Organisation (ISO) proposed a general guideline for diagnostic PCR that requires the presence of an IAC (ISO/DIS 22174).

The IAC should be competitive using the same primer binding sites as the target PCR and clearly distinguishable from the target DNA amplicons by means of length and fluorescence wavelength of the used probe dye in real-time PCR. In summary the characteristics of the control should be as close to the characteristics of the target nevertheless ensuring negligible interference on target detection.

The implementation of an IAC to PCR assays ensures the reliability of negative results; however, this is only true for the enzymatic reaction facilitating target amplification in PCR and for the quantitative and confirmative detection reaction based on the use of fluorescent probes in real-time PCR. However, preliminary methodical steps such as sample preparation and DNA-isolation/purification from the sample matrix are not covered by this kind of control and if at all, checked by external controls. This does not allow for control of single samples and thus negative results imply the possibility of false verification of the pathogen status of the investigated samples, e.g. food.

Therefore an internal sample process control (ISPC) is necessary which should cover all methodical steps which are necessary for reliable quantitative detection of pathogens with conventional or real-time PCR.

For detection of food and waterborne RNA viruses the use of Feline Calcivirus and the bacteriophage MS2 as internal controls has been reported (Mattison et al. (2009) Int. J. Food Microbiol. 132, 73-77; Di et al. (2010) J. Virol. Methods 165, 57-63; Dreier et al. (2005) J. Clin. Microbiol. 43, 4551-4557).

Murphy et al. (2007, Int. J. Food Microbiol. 120, 110-119) discloses a bacterial internal sample process control for the detection of *Listeria monocytogenes* and *Salmonella enterica.* This control is based on a recombinant *E. coli* strain including fragments of the green fluorescence gene (*gfp*) and the *iroB* gene of *S*. *enterica.* The control was not used for quantitative purposes and the underlying gram negative *E. coli* strain will exhibit different characteristics during sample preparation compared to *L*. *monocytogenes*.

Consequently, there is a need for new reliable methods allowing the detection and quantification of the pathogen *Listeria monocytogenes* by real-time PCR in contaminated samples.

Unexpectedly, it has been found that this problem can be solved by using an IAC+, Δ*-prfA L. monocytogenes* EGDe strain as internal process control covering the whole detection process.

### Description of the invention

A first aspect of the present invention is, therefore, a genetically modified bacterium of the species *Listeria monocytogenes,* wherein the genomic locus of the transcriptional factor PrfA has been deleted, characterised in that it comprises on genomic level an artificial sequence that acts as internal amplification control (IAC).

A genetically modified bacterium of the species *Listeria monocytogenes* means a *Listeria monocytogenes* species resulting from altering its genetic material using genetic engineering techniques. Genetic modification comprises the insertion and/or deletion of genes. The deletion of the prfA gene can e.g. be accomplished as disclosed in Böckmann et al. (1996) Mol. Microbiol. 22, 643-653.

The genomic locus of the transcriptional factor PrfA means the nucleic acid sequence encoding for the protein PrfA. This is a sequence of 705 base pairs (Leimeister-Wachter et al., 1990; Proc Natl Acad Sci USA, 87, 8336-40; Glaser et al., 2001; Science, 294, 849-52). The gene is located within the chromosomal region around the listeriolysin gene (*lis*A) of *L. monocytogenes* (GeneID: 987031; http://www.ncbi.nlm.nih.gov/gene/987031#pageTop). Information concerning the transcriptional factor PrfA is also given in Scortti et al. (2007) Microbes and Infection 9, 1196-1207, which is hereby incorporated by reference. PrfA controls the expression of key virulence determinants of *Listeria monocytogenes.* PrfA is a 237-residue protein of 27 kDa. The deletion of the genomic locus of the transcriptional factor PrfA renders the mutant non-pathogen.

According to the present invention internal amplification control (IAC) means an artificial nucleic acid sequence present in the sample tube during PCR reaction and co-amplified with a certain target sequence as defined by Hoorfar et al. (2003) Lett. Appl. Microbiol. 38, 79-80. The presence of an IAC allows to determine false-negative results. The IAC sequence may be any polynucleotide sequence. The size of this sequence may vary dependend on the specific PCR assay and reaction conditions thereof.

The IAC sequence is preferably a single-copy nucleotide sequence, i.e. a nucleotide sequence present only once in the haploid genome of a *Listeria monocytogenes* bacterium.

Preferably, the IAC sequence is a sequence not occurring in *Listeria monocytogenes* or any other species to be expected as naturally occurring within a sample to be investigated, or in the sample matrix itself.

Preferably, the IAC sequence comprises primer binding sequences at the 5' and 3' ends.

According to the present invention it is preferred that the IAC is a competitive IAC, i.e. the IAC of the genetically modified *Listeria monocytogenes* and the target sequence *prfA* of the wild type *Listeria monocytogenes* are amplified with one common set of primers under the same conditions and in the same PCR tube during real time PCR. Therefore, the primer binding sequences of the IAC are particularly preferably identical to the primer binding sequences of the genomic *prfA* locus of wild type *Listeria monocytogenes.*

In a very particularly preferred embodiment of the present invention the artificial IAC sequence is the 100 bp sequence according to SEQ ID NO: 1: 5'-GAT ACA GAA ACA TCG GTT GGC GTA TTC GAA ATG TCC GTT CGG TTG GCG CTA TGA AGA GAT ACG CGG TGG AAC CTG GAA CCT GAT GGC ATC AAG ATT ACA C-3'.
This IAC sequence is disclosed in Rossmanith et al. (2006) Res. Microbiol. 157, 763-771. According to the present invention IAC sequences of more than 80% homology to SEQ ID NO: 1 can be used. Preferably, the sequence homology is more than 90%, more preferably more than 95%.

According to the present invention a genetically modified *Listeria monocytogenes* EGDe strain as specified above is preferred.

The *Listeria monocytogenes* EGDe strain is a *Listeria monocytogenes* serovar 1/2a clinical preparation. This strain is the most widespread model organism in scientific use (GenBank: AL591978; Glaser et al. (2001) Science 294 (5543), 849-52).

A further aspect of the present invention is a genetically modified *Listeria monocytogenes* EGDe strain, wherein the genomic locus of the transcriptional factor PrfA has been deleted, comprising the internal amplification control sequence (IAC) of SEQ ID NO:1 as deposited at the DSMZ (Deutsche Sammlung von Mikroorganismen und Zellkulturen GmbH, Braunschweig) as *Listeria monocytogenes* ΔprfA/+IAC under DSM 23639 on Mai 20, 2010.

The genetically modified bacterium of the species *Listeria monocytogenes* according to the present invention meets the requirements given for a reliable internal sample process control. It is as closely related to wild-type *Listeria monocytogenes* as possible. It does not interfere with the main detection reaction by means of real-time PCR and the performance of the underlying chemical reaction for the detection of the control is equal to the main reaction. Additionally, the application of Listeria monocytogenes cells as ISPC permit to cover the whole progression of the necessary methods included in food pathogen detection from sample preparation to detection using real-time PCR.

Another aspect of the present invention is the use of genetically modified *Listeria monocytogenes* as specified above for detecting and determining qualitatively and / or quantitatively the occurrence of wild type *Listeria monocytogenes* in a sample suspected to be contaminated with said micro-organism.

Preferably, the sample is a food sample, a body fluid, in particular blood, plasma or serum, water or a tissue sample.

Exemplary samples include, but are not limited to, food (e.g. milk of cows, ewes, nanny goats, mares, donkeys, camels, yak, water buffalo and reindeer, milk products, meat of beef, goat, lamb, mutton, pork, frog legs, veal, rodents, horse, kangaroo, poultry, including chicken, turkey, duck, goose, pigeon or dove, ostrich, emu, seafood, including finfish such as salmon and tilapia, and shellfish such as molluscs and crustaceans and snails, meat products, plant products, seeds, cereals from grasses, including maize, wheat, rice, barley, sorghum, and millet, cereals from non-grasses, including buckwheat, amaranth, and quinoa, legumes, including beans, peanuts, peas, and lentils, nuts, including almonds, walnuts, and pine nuts, oilseeds, including sunflower, rape and sesame, vegetables like root vegetables, including potatoes, cassava and turnips, leaf vegetables, including amaranth, spinach and kale, sea vegetables, including dulse, kombu, and dabberlocks, stem vegetables, including bamboo shoots, nopales, and asparagus, inflorescence vegetables, including globe artichokes, broccoli, and daylilies, and fruit vegetables, including pumpkin, okra and eggplant, fruits, herbs and spices, whole blood, urine, sputum, saliva, amniotic fluid, plasma, serum, pulmonary lavage and tissues, including but not limited to, liver, spleen, kidney, lung, intestine, brain, heart, muscle, pancreas and the like. The skilled artisan will appreciate that lysates, extracts or (homogenized) material obtained from any of the above exemplary samples or mixtures of said exemplary samples or compositions comprising one or more of said exemplary samples are also samples within the scope of the invention.

Particularly preferred is a food sample.

The food sample is preferably a milk product, preferably milk, in particular raw milk, milk powder, yoghurt, cheese or ice cream, a fish product, preferably raw fish, a meat product, preferably raw meat, meat rinse or sausages, salad rinse, chocolate, egg or egg products, like mayonnaise.

Particularly preferred food samples used in the method according to the present invention are samples which are usually known to comprise potentially pathogenic *Listeria monocytogenes,* e.g. cheese.

Preferably, the genetically modified *Listeria monocytogenes* as specified above is used as internal sample process control (ISPC) for a real-time PCR based assay.

According to the present invention an ISPC is a model organism added to the original sample prior to sample preparation. An ISPC provides a measure for the efficiency of the whole analytical chain from sample preparation to target molecule detection and covers all methodical steps which are necessary for reliable quantitative detection of pathogens with conventional or real-time PCR.

A further aspect of the present invention is a method for detecting and determining the occurrence of wild type *Listeria monocytogenes* in a sample suspected to be contaminated with said pathogenic micro-organism, comprising the steps:
(a) adding to said sample a predefined amount of cells of the genetically modified *Listeria monocytogenes* bacterium as specified above,
(b) incubating the sample with an extraction solution,
(c) isolating the DNA by standard methods;
(d) applying real-time PCR, thereby using (i) primers specific for the genomic *prfA* locus of wild type *Listeria monocytogenes* and the IAC sequence of genetically modified *Listeria monocytogenes* as specified above; and (ii) a fluorescent labelled oligonucleotide probe that is able to specifically hybridize with said *prfA* locus and a fluorescent labelled oligonucleotide probe that is able to specifically hybridize with said IAC sequence,
(e) determining qualitatively and / or quantitatively fluorescent signals generated by step (d), and
(f) determining and / or calculating from step (e) the presence and / or the amount of the wild type *Listeria monocytogenes* cells in the original sample suspected to be contaminated with said micro-organism.

In step (a) a predefined amount of cells of the genetically modified *Listeria monocytogenes* bacterium as specified above is added to the sample. The amount added to the sample depends on the sample size. Preferably, an amount of 25 to 100000 CFU (colony forming units) of the genetically modified *Listeria monocytogenes* is added to a sample of e.g. 25 g. Particularly preferred is an amount of 100 to 5000 CFU per 25 g.

Colony forming unit (CFU) is a measure of viable cells in a sample. It can be determined by culture methods, e.g. by evenly spreading the sample on a bacterial culture gel. Incubation at suitable culture conditions results in the formation of colonies. The number of the colonies represents the number of colony forming units in the sample. Alternatively the cell count is determinable by microscopic count of fluorescent stained cells, e.g. using the Live/Dead® *Bac*Light™ Bacterial Viability Kit (Molecular Probes, Willow Creek, OR, USA) or compareable commercial methods.

The extraction solution used in step (b) is an aqueous solution or a buffer solution. It typically has a pH value greater than 5 and lower than 9, preferably greater than 6 and lower than 8, more preferably between 6.5 and 7.5. The extraction solution may additionally comprise up to 20% of one or more water-miscible organic solvents.
The buffer which may be used in the method of the present invention is preferably selected from the group of phosphate buffer, phosphate buffered saline buffer (PBS), 2-amino-2-hydroxymethyl-1, 3-propanediol (TRIS) buffer, TRIS buffered saline buffer (TBS) and TRIS/EDTA (TE).

In one embodiment of the present invention the extraction solution further comprises MgCl₂ and/or an ionic liquid. The MgCl₂ - if present - is typically present in concentrations between 0.05 and 3 M, preferably between 0.1 and 2 M, more preferably between 0.3 and 1 M.

In another embodiment of the present invention the extraction solution further comprises at least one chaotrope and at least one detergent.

The ionic liquid - if present - is typically present in concentrations between 0.5 and 20% by weight, preferably between 1 and 10 % by weight, based on the weight of mixture. The ionic liquid can be one ionic liquid or a mixture of two or more ionic liquids. In a preferred embodiment, the extraction solution comprises either MgCl₂ or ionic liquid.

Ionic liquids used in the present invention are ionic species which consist of an organic cation and a generally inorganic anion. They do not contain any neutral molecules and usually have melting points below 373 K. Review articles on ionic liquids are, for example, R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flussigkeiten - neue Lösungen fur die Ubergangsmetallkatalyse" [Ionic Liquids - Novel Solutions for Transition-Metal Catalysis], Angew. Chem., 112 (2000), 3926-3945; T. Welton "Room temperature ionic liquids. Solvents for synthesis and catalysis", Chem. Rev., 92 (1999), 2071-2083 or R. Hagiwara, Ya. Ito "Room temperature ionic liquids of alkylimidazolium cations and fluoroanions", J. Fluorine Chem., 105 (2000), 221-227).

In general, all ionic liquids of the general formula K⁺A⁻ known to the person skilled in the art, in particular those which are miscible with water, are suitable in the method according to the invention.

If an ionic liquid or MgCl₂ is present, in a further preferred embodiment, the extraction solution comprises no detergent, that means no anionic, zwitterionic or non-ionic detergent like sodium dodecylsulfate, CHAPS, Lutensol AO-7, is added to the extraction solution.

The term "chaotrope" as used herein, refers to a substance that causes disorder in a protein or nucleic acid by, for example, but not limited to, altering the secondary, tertiary or quaternary structure of a protein or a nucleic acid while leaving the primary structure intact. Exemplary chaotropes include, but are not limited to, guanidine hydrochloride (GuHCl), guanidinium thiocyanate (GuSCN), sodium thiocyanate (KSCN), sodium iodide, sodium perchlorate, urea, and the like. Descriptions of chaotropes and chaotropic salts can be found in, for instance, in K. Hamaguchi et al. (Proc. Natl. Acad. Sci. (1962) 62:1129-1136)

As used herein, the term "detergent" refers to molecules having lipophilic as well as hydrophilic (i.e. amphiphilic) characteristics. A detergent according to the present invention may comprise, for instance, a fatty acid residue and a hydrophilic (e.g. anionic or cationic) part.

Furthermore, it is possible to add to the extraction solution one or more additional substances like destabilizing agents or biopolymer degrading enzymes which help to degrade substances present in specific samples. One example is the addition of starch degrading enzymes for food sample comprising high amounts of collagen and/or starch.

The incubation is typically performed at temperatures between 18°C and 50°C, preferably between 25°C and 45°C, more preferably between 30°C and 42°C.

The sample is typically incubated with the extraction solution for a time between 10 minutes and 6 hours, preferably between 20 minutes and 1 hour.

In order to facilitate the dissolution of the sample, said sample can be, for instance, homogenized using a stomacher prior its incubation with the extraction solution. The dissolution is further supported and/or accelerated when the mixture is agitated during the incubation.

Further extraction methods can for example be found in Brehm-Stecher et al. (2009) Journal of Food Protection 72: 1774-1789.

According to the present invention, the DNA of the bacterial material is isolated in step (c). Various methods known in the art may be employed to extract DNA, e.g. the methods disclosed in Sambrook et al. (1989) Molecular cloning: a laboratory manual, 2nd ed. Cold Spring Harbor Laboratory Press, Cold Spring Harbor, N.Y..
In general, DNA extraction comprises cell lysis and DNA purification by either precipitation or specific binding on various substrates e.g. silica.

Cell lysis may be accomplished by standard methods, e.g. by enzymatic methods, bead methods, sonication, detergent methods or combinations thereof. Preferably, detergent methods are employed.
Suitable enzymes for enzymatic cell lysis are, e.g., lysozyme, lysostaphin, zymolase, cellulase, mutanolysin, glycanases, proteases, mannase.

Beads suitable for cell disruption are of glass, ceramic, zirconium, or steel. After the addition of beads to the cells agitation by stirring or shaking of the mix is applied. Agitation can for example be applied by a common laboratory vortex mixer or in a specially designed clamp.

According to the present invention, a further method for cell lysis is sonication. This method involves ultrasound application (typically 20-50 kHz) onto the sample.

Detergent-based cell lysis results in the disruption of the lipid barrier surrounding cells. Suitable detergents may be chosen from non-ionic, zwitterionic and ionic detergents, e.g. CHAPS, Triton® X or TWEEN®. Preferably, ionic detergents are used. An example for a suitable ionic detergents is SDS.

In addition to the choice of detergent, other important considerations for optimal cell lysis include the buffer, pH, ionic strength and temperature:

The lysis solution typically has a pH value greater than 5 and lower than 9, preferably greater than 6 and lower than 8, more preferably between 6.5 and 7.5.
The buffer which may be used is preferably selected from the group of phosphate buffer, phosphate buffered saline buffer (PBS), 2-amino-2-hydroxymethyl-1, 3-propanediol (TRIS) buffer, TRIS buffered saline buffer (TBS) and TRIS/EDTA (TE).

Optionally, one or more chelating agents can be added to the lysis solution to sequester divalent cations. Suitable chelators are, e.g., EDTA (ethylenediamine tetraacetic acid), EGTA (ethylene glycol tetraacetic acid) or ethylenediamine. Preferably, EDTA is used.

The above mentioned cell lysis methods are typically followed by centrifugation in order to separate the DNA from the cellular material. A skilled person can easily determine the parameters for centrifugation. Typically, centrifugation is carried out as dislosed in Sambrook et al. (1989) Molecular cloning: a laboratory manual, 2nd ed. Cold Spring Harbor Laboratory Press, Cold Spring Harbor, N.Y.

After cell lysis the DNA is usually precipitated by adding an alcohol, preferably ethanol or isopropanol.

Additionally, the DNA can be provided in a form which is suitable for amplification using a commercially available DNA isolation kit, such as the NucleoSpin® tissue kit and the support protocol for Gram-positive bacteria (Machery-Nagel, Düren, Germany) or the Nexttec® kit for genomic DNA from bacteria (Nexttec GmbH Biotechnologie, Leverkusen, Germany).

In a further embodiment of the present invention the bacterial material is separated prior to DNA isolation in step (c). This further step is particularly preferred since it allows for achieving a higher concentration of DNA in the resulting PCR sample. Separation of the bacterial material can be accomplished by any known method, like centrifugation, filtration, dielectrophoresis and ultrasound or affinity binding, e.g. using antibodies, lectins, viral binding proteins, aptamers or antimicrobial peptides (AMP) which are preferably immobilized on beads. Preferably, the cells are isolated by filtration or centrifugation, most preferred by centrifugation. Filtration of the extracted sample is in particular required when the complex sample comprises material which is hardly or not extractable with the method of the present invention. Typically these materials comprise starch and/or fibres. However, the preferred method for isolation the cells from the extraction mixture is centrifugation.

The incubation step may - depending on the sample matrix - be repeated once or several times, e.g. twice, three times, four times, five times or ten times. Between these incubation steps the bacterial material and the remnant sample matrix may be separated from the supernatant by e.g. centrifugation.

After the separation of the bacterial material the cells are preferably washed with water, a buffer solution and/or detergent comprising solutions. The wash step may be repeated several times.

In step (d) PCR, preferably real-time PCR is applied. Real-time PCR is a method for detecting and measuring products generated during each cycle of a PCR, which are proportionate to the amount of template nucleic acid prior to the start of PCR. The information obtained, such as an amplification curve, can be used to quantitate the initial amounts of template nucleic acid sequence.
The detection is preferably based on monitoring fluorescence at every cycle at a set temperature. Fluorescence is usually monitored using an optical device to collect the data at specific excitation and emission wavelengths for the particular fluorescent dye present in the sample. The cycle at which the fluorescence from a sample crosses the threshold for detection of fluorescence above background is called the cycle threshold, Ct, and allows the quantification of the starting template.

The PCR conditions are not particularly restricted but optimal conditions may be selected for each PCR apparatus. For example, the following conditions may be used:
- Thermal denaturation of double-stranded DNA to single-stranded DNA: Heating is generally made at about 90-98°C, preferably at about 92-96°C, generally for about 3 seconds to 1 minute, preferably for about 30 seconds to 1 minute.
- Annealing: Heating is made generally at about 40-70°C, preferably at 55-65°C, generally for about 5 seconds to 2 minutes, preferably for about 30 seconds to 90 seconds.
- DNA elongation reaction: Heating is made generally at about 60-75°C, preferably at about 70-74°C, generally for about 10 seconds to 3 minutes, preferably for about 30 seconds to 2 minutes.
- Mg ion concentration in the reaction liquid: Generally about 1-5 mM, preferably about 1.5-3.5 mM.

This reaction is typically carried out in about 20-50 cycles, preferably in about 45 cycles, whereby the target DNA can be amplified to a detectable level.
Any commercial real-time PCR apparatus can be used.

According to step (d)(i) of the method of the present invention primers specific for the genomic *prfA* locus of wild type *Listeria monocytogenes* and the IAC sequence of genetically modified *Listeria monocytogenes* as defined above are used.

In general, the term "primer" refers to a short nucleic acid molecule, such as a DNA oligonucleotide of 9 nucleotides or more in length, that is complementary to a section along a strand of the target nucleic acid, i.e. the genomic *prfA* locus and the IAC sequence, wherein the purpose of the primer is to initiate the nucleic acid replication of a longer nucleic acid along the strand. According to the present invention primers of 15 to 40 nucleotides are preferred. In the present invention the term "primers" is used for the primer pair, flanking the targeted sequence to be amplified.

Preferably, the primers used in step (d)(i) are Lip1 and Lip2.
The sequence of Lip1 is: 5'-GAT ACA GAA ACA TCG GTT GGC-3' (SEQ ID NO: 2) and the sequence of Lip2 is: 5'-GTG TAA TCT TGA TGC CAT CAG G-3' (SEQ ID NO: 3).

According to step (d)(ii) of the method of the present invention a fluorescent labelled oligonucleotide probe that is able to specifically hybridize with said *prfA* locus and a fluorescent labelled oligonucleotide probe that is able to specifically hybridize with said IAC sequence are used.

According to the present invention the term "specifically hybridize" means to detectably and specifically bind. Polynucleotides, oligonucleotides and fragments thereof selectively hybridize to nucleic acid strands under hybridization conditions that minimize appreciable amounts of detectable binding to non-specific nucleic acids.

A fluorescent labelled oligonucleotide is an oligonucleotide exhibiting a typically covalently attached fluorophor. A fluorophor is a chemical compound, which when excited by exposure to a particular wavelength of light, emits light (fluoresces), for example at a different wavelength of light.

Preferably, the oligonucleotide probes that are able to specifically hybridize with said *prfA* locus and said IAC sequence exhibit different fluorophors emitting light of different wavelengths. This allows the detection of one probe independently from the other.

The fluorescent labelled probes typically used for real time PCR are e.g. LightCycler (hybridisation) probes, molecular beacons or hydrolysis probes (also called TaqMan® probes). Preferably, hydrolysis probes are used.

LightCycler probes utilize the technique of fluorescence resonance energy transfer (FRET). For this method two different oligonucleotide probes bound to a FRET donor and a FRET acceptor, respectively, are used for each target sequence. These probes bind side by side to the target sequence, bringing the fluorophors in proximity.

Molecular beacons (Tyagi et al., Nat. Biotechnol. 14:303-8, 1996) are oligonucleotide probes bound to a reporter fluorophor and a quencher. The nucleotides on the 5' end are complementary to the nucleotides on the 3' end, forming a stem loop structure. Because of the proximity of the fluorophor and the quencher no fluorescence is observed. Hybridization of the probe with the target sequence during real-time PCR leads to an increase in the reporter-quencher distance resulting in fluorescence of the reporter.

Preferably, hydrolysis probes (TaqMan® probes) are used (Lee et al., Nucleic Acids Res. 21:3761-6, 1993). These probes utilize as well the technique of fluorescence resonance energy transfer (FRET). The probes exhibit a fluorescent reporter at one end and a quencher of fluorescence at the opposite end. Because of the close proximity of the reporter to the quencher detection of the reporter fluorescence is suppressed. During the annealing stage of the PCR both primers and the probe anneal to the DNA target. Polymerization of a new DNA strand leads to the degradation of the probe by the 5'-3' exonuclease activity of the polymerase and physical separation of the fluorescent reporter from the quencher, resulting in an increase in fluorescence. Fluorescence can be detected and measured in the real-time PCR thermocycler, and its geometric increase corresponding to exponential increase of the product is used to determine the threshold cycle (Ct) in each reaction (see below).

Exemplary reporters include, but are not limited to: 6-carboxyfluorescein; carboxyfluorescein (FAM); boron dipyrromethene difluoride (BODIPY); acridine, stilbene, 6-carboxy-fluorescein (HEX), TET (Tetramethyl fluorescein), 6-carboxy-X-rhodamine (ROX), Rhodamine-6G, Texas Red, 2',7'-dimethoxy-4',5'-dichloro-6-carboxyfluorescein (JOE), Cy®3, Cy®5, VIC® (Applied Biosystems), LC Red 640, LC Red 705, Texas Red, Yakima Yellow®, as well as derivatives thereof. Preferably, a reporter selected from FAM and HEX is used in the present invention.

Exemplary quenchers include, but are not limited to Black Hole Quenchers (WO 01/86001 A1) as BHQ1^{™} and BHQ2^{™}, MGB (Minor-groove-binder, EP 0819133 B1), N,N,N',N'-tetramethyl-6-carboxyrhodamine (TAMRA), Eclipse® Dark Quencher, DABCYL, DABSYL, DDQ I and DDQ II According to the present invention, preferably used quenchers are MGB or BHQ1^{™}.

A person skilled in the art can easily chose a suitable reporter-quencher combination. Typically, the absorption spectrum of the quencher needs to have a good overlap with the emission spectrum of the reporter in order to allow for optimal quenching.

Preferably, the fluorescent labelled probe hybridizing with the IAC sequence of step (d)(ii) is pLucLm4 (SEQ ID NO:4) as disclosed in Rossmanith et al. (2006) Res. Microbiol. 157, 763-771. The fluorescent labelled probe hybridizing with the *prfA* locus is preferably LipProbe (SEQ ID NO:5).

The fluorescent labelled probe pLucLm4 exhibits preferably HEX as reporter fluorophor and BHQ1^{™} as quencher.

The fluorescent labelled probe LipProbe exhibits preferably FAM as reporter and MGB as quencher.

According to the present invention the fluorescent signals generated by step (d) are qualitatively and / or quantitatively determined in step (e).

The detection is preferably based on monitoring fluorescence at every cycle at a set temperature. Fluorescence is usually monitored using an optical device to collect the data at specific excitation and emission wavelengths for the particular fluorophors present in the sample.

In step (f) the presence and / or the amount of wild type *Listeria monocytogenes* cells in the original sample suspected to be contaminated with said micro-organism is determined and / or calculated from step (e).

The presence of wild type *Listeria monocytogenes* cells in the original sample is typically determined by qualitatively determining the fluorescent signals in step (e).

The amount of wild type Listeria monocytogenes cells in the original sample is typically calculated comprising the following steps:
- Calculating the initial amount of DNA by means of DNA copies of the genetically modified *Listeria monocytogenes* and wild-type *Listeria monocytogenes* by using the Ct method (Threshold method) based on the respective fluorescence signal after real-time PCR amplification. The threshold method is based on the comparsion of the respective signal of the investigated sample with a calibration standard (Kaltenböck et al. (2005), Advances in Clinical Chemistry 40: 219-259).
- Calculating the loss during the analytical procedure (comprising sample preparation, DNA purification and isolation and real-time PCR according to steps (b), (c) and (d) of the method of the present invention) based on the known value of initial cells of genetically modified *Listeria monocytogenes* within the sample and the value of cells as obtained by the Ct method after real-time PCR.
- Calculating the number of wild type *Listeria monocytogenes* cells present in the sample suspected to be contaminated with said microorganism based on the calculated loss of genetically modified *Listeria monocytogenes* cells during the analytical procedure.

The method of the present invention is advantageous since the use of *Listeria monocytogenes* as an internal sample process control (ISPC) for molecular biological pathogen detection provides most similarity with the target organism and therefore most applicability for bacterial pathogen detection. Even a nearly related species such as *Listeria innocua* would lead to a non-competitive internal control requiring a second primer pair during real-time PCR. The simple use of a DNA based ISPC derived from an existing IAC, from the very beginning of the detection process, would as well not fulfil the prerequisite of most similarity of control and target. Besides, most of the DNA would get lost during the various methodical steps before PCR detection.

A further aspect of the present invention is a kit for use in an assay for detecting and determining wild type *Listeria monocytogenes* in a sample suspected to be contaminated with said micro-organism, comprising at least in one or more packages
(i) genetically modified *Listeria monocytogenes* as specified above;
(ii) the primers specific for the genomic *prfA* locus of wild type *Listeria monocytogenes* and the IAC sequence of genetically modified *Listeria monocytogenes* as specified above; and
(iii) a fluorescent labelled oligonucleotide probe that is able to specifically hybridize with said *prfA* locus and a fluorescent labelled oligonucleotide probe that is able to specifically hybridize with said IAC sequence.

Preferably, the primers of the kit are Lip1 (SEQ ID NO:2) and Lip2 (SEQ ID NO:3).

The fluorescent labelled probes are preferably pLucLm4 (SEQ ID NO:4) for detecting the IAC sequence and LipProbe (SEQ ID NO:5) for detecting the *prfA* locus.

According to the present invention the kit may additionally comprise an extraction solution.

Another aspect of the present invention is a method for manufacturing a genetically modified *Listeria monocytogenes* bacterium as specified above comprising the steps:
(a) deleting the genomic locus of the transcriptional factor PrfA of the bacterial species *Listeria monocytogenes,*
(b) cloning of a vector comprising an IAC,
(c) transforming the vector of step (b) into the bacterial species of step (a).

The deletion of the genomic locus of PrfA according to step (a) is accomplished by techniques known in the art and can for example be accomplished as disclosed in Böckmann et al. (1996) Mol. Microbiol. 22, 643-653. In principle, a knock-out plasmid is constructed. This plasmid is transformed into *Listeria monocytogenes* cells. Crossing-over results in the excision of the *prfA* gene. The resulting organism is also referred to as Δ-*prfA Listeria monocytogenes.*

Optimal culture conditions for *Listeria monocytogenes* bacteria can easily be determined by a skilled person. Typically, the bacteria are grown in TSB-Y (Trypton soya broth with yeast extract) at 37°C with aeration.

In step (b) a vector comprising the IAC is produced.
Adequate amounts of the IAC sequence for cloning are typically produced by amplification of the sequence by conventional PCR (Rossmanith et al. (2006) Res. Microbiol. 157, 763-771). The primers used for PCR typically comprise restriction sites for special restriction enzymes allowing for later integration into the vector. For an IAC sequence according to SEQ ID NO:1 the modified primers LipBam (SEQ ID NO:6) and LipSal (SEQ ID NO:7) comprising the restriction sites BamHI and SalI, respectively, are preferably used.
The sequence of LipBam is: 5'GCG CGG ATC CGA TAC AGA AAC ATC GGT TGG C'3 (SEQ ID NO:6).
The sequence of LipSal is 5'GCG CGT CGA CGT GTA ATC TTG ATG CCA TCA GG'3 (SEQ ID NO:7).

After purification of the amplification product restriction digestion, dephosphorylation and ligation with a suitable integration vector are performed.

Restriction digestion is typically performed by incubation of the amplification product with commercially available restriction enzymes corresponding to the restriction sites of the primers used for PCR. Preferably, BamHI and SalI are used.

Dephosphorylation of the IAC fragments can be accomplished by incubation with a suitable phosphatase, e.g. Fast AP^{™} Thermosensitive Alkaline Phosphatase (Fermentas). A suitable integration vector can easily be chosen by a skilled person. The vector comprises a gene sequence giving resistance to an antibiotic that the intended recipient strain of bacteria is sensitive to (e.g. chloramphenicol) in order to allow for later selection of positive clones. Preferably, a phage insertion vector, e.g. pPL1 or pPL2 as disclosed in Lauer et al. (2002) J. Bacteriol. 184, 4177-4186 is used. The phage insertion vector pPL2 (6123 bp) is particularly preferred since it provides stable single copy insertion into the genome of *Listeria monocytogenes.*
Adequate amounts of the insertion vector can be produced by amplifying the vector in a bacterium, e.g. in *E.coli* TOP10F', and subsequent isolation, restriction digestion and dephosphorylation as described above. Typically, the restriction sites can be found in a multiple cloning site (MCS) of the vector.

Ligation is typically performed by the addition of a DNA ligase, e.g. T4 DNA ligase available from Fermentas.

The resulting vector is transformed into a suitable bacterium, e.g. *E.coli* TOP10F' for amplification and for the selection of positive clones. This selection is typically accomplished by plating the bacteria on a medium comprising the above mentioned antibiotic.

In step (c) the vector of step (b) is transferred into the *Listeria monocytogenes* cells of step (a). The transformation can be carried out using the various methods well-known in the state of the art. Examples are transformation by heat shock or electroporation. Preferably, transformation is effectuated by electroporation.

Transformation by heat shock is accomplished by chilling the cells in the presence of divalent cations such as Ca²⁺ (in CaCl₂) or Rb²⁺ (RbCl₂) preparing the cell membrane to become permeable to plasmid DNA. Cells are incubated on ice with the DNA and then briefly heat shocked (typically at 41 to 43°C for 30 to 120 seconds).

For electroporation the cells are briefly shocked with an electric field of 10-25 kV/cm. Preferably, electroporation is accomplished according to Park et al. (1990) Gene 94, 129-132.

The resulting organism can be tested upon successful cloning. Successful insertion of the vector into the Δ-*prfA Listeria monocytogenes* genome can be tested by amplification of the 499 bp fragment by PCR (according to Lauer et al. (2002) J. Bacteriol. 184, 4177-4186) and by sequencing.

Single copy insertion of the IAC into the genome of Δ-*prfA Listeria monocytogenes* can be verified by comparison of the genomic DNA amounts of transformed *Listeria monocytogenes* measured by UV/VIS spectroscopy with the data after real-time PCR in comparison to wild-type data, based on the known size of the *Listeria* genome (Nelson et al.(2004) Nucleic Acids Res. 32, 2386-2395).

The present invention is further illustrated by the following figures and examples, however, without being restricted thereto.

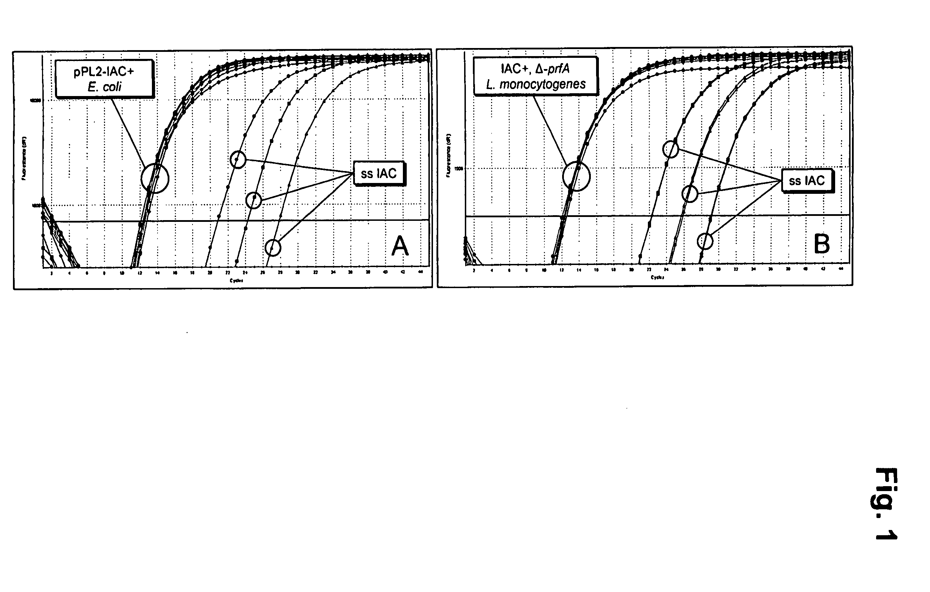

**Figure 1****. Confirmation of pPL2-IAC, *E. coli* and IAC+, Δ-*****prfA L.** monocytogenes* EGDe (according to Examples 1.4 and 1.6). (A) Real-time PCR amplification of pPL2-IAC, *E. coli* clones after transformation and plasmid isolation targeting the artificial IAC within the plasmid. The amplification plots on the left side include the plasmid preparation as target. The amplification plots on the right side (ss IAC) represent the calibration function (Efficiency.: 96.8 %; Rsq: 0.999). (B) Real-time PCR amplification of IAC+, Δ*-prfA L. monocytogenes* EGDe clones targeting the artificial IAC within the genome for confirmation of integration of the vector pPL2-IAC into the *Listeria* genome (Eff.: 96.0 %; Rsq.: 0.999). The amplification plots on the left side include genomic DNA of four IAC+, Δ*-prfA L. monocytogenes* EGDe clones as target.

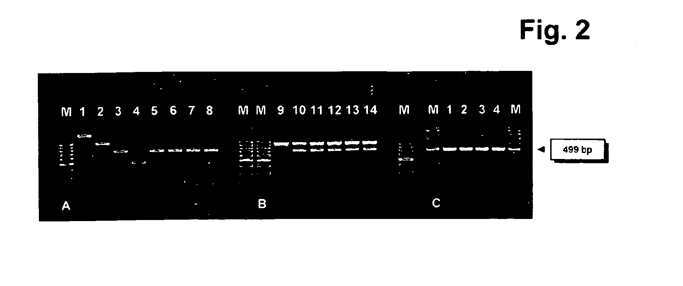

**Figure 2****. Confirmation of positive integration of pPL2-IAC into the *Listeria* genome and confirmation of the resulting IAC+, Δ*-prfA L. monocytogenes* EGDe strain by conventional PCR** (according to Example 1.6.). (A) Agarose gel of the cloned strains, confirmed as *L*. *monocytogenes.* M, Marker; 1, *L. ivanovii, L. seeligeri and L. welshimeri; 2, L. innocua;* 3, *L. monocytogenes;* 4, *L. grayi*; 5-8, Four IAC+, Δ*-prfA L. monocytogenes* EGDe clones. (B) Confirmation of the IAC+, Δ*-prfA L. monocytogenes* EGDe amplifying the 16S rRNA gene specific for all *Listeria* spp. and the *hly* gene specific for *L. monocytogenes.* 9, *L*. spp.; 10, *L. monocytogenes;* 11-14, Four IAC+, Δ*-prfA L. monocytogenes* EGDe clones. (C) Confirmation of the integration of the pPL2-IAC into the genome of the Δ*-prfA L. monocytogenes* EGDe strain. 1-4, Four IAC+, Δ*-prfA L. monocytogenes* EGDe clones. The resulting 499 bp fragment after PCR indicates integration. PCR products are separated in a 1% agarose gel and stained with ethidium bromide.

The entire disclosures of all applications, patents, and publications cited above and below are hereby incorporated by reference.

### 1. Examples:

The following examples describe practical applications of the invention.

**1.1. Bacterial strains and culture conditions.** *L. monocytogenes* EGDe (1/2a, internal number 2964) is part of the collection of bacterial strains at the Institute of Milk Hygiene, Milk Technology and Food Science, Department of Veterinary Public Health and Food Science, University of Veterinary Medicine, Vienna, Austria (IMML). Δ*-prfA L. monocytogenes* EGDe (1/2a) is part of the collection of bacterial strains at the Department of Microbiology, Theodor Boveri Institute, University of Würzburg, Würzburg, Germany. Electro-competent *E*. *coli* TOP10F' are available at Invitrogen (Carlsbad, CA, USA). All bacterial strains are maintained at - 80°C using MicroBank™ technology (Pro-Lab Diagnostics, Richmont Hill, Canada).

Measurement of the optical density of bacterial cultures is performed at 600 nm (OD₆₀₀) in duplicate with an HP 8452 spectrophotometer (Hewlett Packard, Paolo Alto, CA, USA). Selective plating of *L. monocytogenes* EGDe wt and IAC+, Δ*-prfA L. monocytogenes* EGDe is performed on RAPID' L.mono (Bio-Rad laboratories GmbH, München, Germany), OCLA (Oxoid Chromogenic Listeria agar; Oxoid, Hampshire, UK), PALCAM (Solabia Biokar Diagnostics, Pantin Cedex, France) and blood agar (Biomerieux, Marcy l'Etoile, France) whether by streaking 100µl bacterial culture to the plates or by loop technique.
Enumeration of bacterial suspensions is performed using the plate count method or the Live/Dead^{®} *Bac*Light^{™} Bacterial Viability Kit (Molecular Probes, Willow Creek, OR, USA) according to manufacturer's instructions.

**1.2. Oligonucleotides, plasmids and enzymatic reactions.** The sequences of oligonucleotides and plasmids are presented in Table 1. Primers for conventional and real-time PCR and the HEX-labelled probe pLuclm4 are available from MWG Biotech (Ebersberg, Germany); the MGB-modified Lip-probe from Applied Biosystems (Foster City, CA, USA). The artificial 100 bp target for the IAC is synthesized by VBC Genomics (Vienna, Austria). The pPL2 phage insertion vector according to Lauer et al. (2002) J. Bacteriol. 184, 4177-4186.

**TABLE 1.**

| Oligonucleotide and primer sequences and organisms | | | |
|---|---|---|---|
| Oligonucleotides | | Sequence/Label | Ref. / Source |
| Lip 1 | Forward primer*^{a}* | 5'-GAT ACA GAA ACA TCG GTT GGC-3' | D'Agostino et al., 2004 |
| Lip 2 | Reverse primer*^{a}* | 5'-GTG TAA TCT TGA TGC CAT CAG G-3' | D'Agostino et al., 2004 |
| Lip Bam | Forward primer*^{b}* | 5' GCG CGG ATC CGA TAC AGA AAC ATC GGT TGG C'3 | SEQ ID NO: 6 |
| Lip Sal | Reverse primer*^{c}* | 5'GCGCGT CGA CGT GTA ATC TTG ATG CCA TCA GG'3 | SEQ ID NO: 7 |
| LipProbe | Probe binding *prfA* locus*^{a}* | FAM-CAG GAT TAA AAG TTG ACC GCA-MGB | Rossmanith et al, 2006. SEQ ID NO: 5 |
| pLucLm 4 | Probe binding artificial target | HEX-TTC GAA ATG TCC GTT CGG TTG GC-BHQI | Rossmanith et al., 2006, SEQ ID NO: 4 |
| IAC | Artificial target | | Rossmanith et al., 2006, SEQ ID NO: 1 |
| NC 16 | Confirmation of pPL2 insertion | 5'-GTC AAA ACA TAC GCT CTT ATC-3' | Lauer et al., 2002 |
| PL 95 | Confirmation of pPL2 insertion | 5'-ACA TAA TCA GTC CAA AGT AGA TGC-3' | Lauer et al., 2002 |
| Sequ. 1 (NC 16) | Confirmation of pPL2 insertion | Sequence included in Table 1A | This work |
| Sequ. 2 (PL 95) | Confirmation of pPL2 insertion | Sequence included in Table 1A | This work |

| Species / Plasmid | Strain | | |
|---|---|---|---|
| *L monocytogenes* wt | EGDe (1/2a) | Wild type strain detected byprfA real-time PCR | IMML*^{d}* Strain Nr. 2964 |
| *L monocytogenes* Δ*target* | EGDe (1/2a) Δ*prfA* | *L monocytogenes* EGDe strain with total deletion of the *prfA* locus | Böckmann et al., 1996 |
| *E. coli* TOPIOF' | - | Chemically competent *E. coli* | Invitrogen |
| pPL2 Phage insertion ve ctor | *L monocytogenes* specific | PSA intergration site. Stable single copy mtegants: -10⁻⁴/donor cell. | Lauer et al., 2002 |

| | | | |
|---|---|---|---|
| *a* Specific to *L. monocytogenes* wt *b* Primer containing Bam HI restriction site. *c* Primer containing Sal 1 restriction site. *d* IMML: Institute of Milk Hygiene, Milk Technology and Food Science, Univasity of Veterinary Medicine | | | |

**TABLE 1A. Sequences and blast reports of the analysis of the amplification products of the PCR according to Lauer et al., 2002**

| Name / Primer | Blast report | Reference*^{a}* | Sequence*^{b}* |
|---|---|---|---|
| Sequence 1 Clone 1/NC16 | Identities = 210/210 (100%), | emb AJ417449.2 Shuttle integration vector pPL2 | |
| | Gaps = 0/210 (0%), | | |
| | Strand=Plus/Plus | | |
| | Identities=267/268 (99%), | emb AL591978.1 Listeria monocytogenes strain EGD, complete genome, segment 6/12 | |
| | Gaps = 1/268 (0%), | | |
| | Strand=Plus/Plus | | |
| Sequence 1 Clone 1/NC 16 | Identities = 210/210 (100%), | emb AJ4174492 Shuttle integration vector pPL2 | |
| | Gaps = 0/210 (0%), | | |
| | Strand=Plus/Plus | | |
| | Identities = 261/261 (100%), | emb AL591978.1 Listeria monocytogenes strain EGD, complete genome, segment 6/12 | |
| | Gaps = 0/261 (0%), | | |
| | Strand=PHs/Plus | | |
| Sequence 1 Clone 1/NC 16 | Identities = 209/210 (99%), | emb AJ4174492 Shuttle integration vector pPL2 | |
| | Gaps = 1/210 (0%), | | |
| | Strand=Plus/Plus | | |
| | Identities = 267/268 (99%), | emb AL591978.1 Listeria monocytogenes strain EGD, complete genome, segment 6/12 | |
| | Gaps = 1/268 (0%), | | |
| | Strand=Plus/Plus | | |
| Sequence 1 Clone 1/NC 16 | Identities = 211/211 (100%), | emb AJ417449.2 Shuttle integration vector pPL2 | |
| | Gaps = 0/211 (0%), | | |
| | Strand=Plus/Plus | | |
| | Identities = 266/268 (99%). | emb AL591978.1 Listeria monocytogenes strain EGD, complete genome, segment 6/12 | |
| | Gaps = 2/268 (0%), | | |
| | Strand=Plus/Plus | | |
| Sequence 1 Clone 1/PL95 | Identities = 172/176 (97%), | emb AJ417449.2 Shuttle integration vector pPL2 | |
| | Gaps = 3/176 (1%), | | |
| | Strand=Plus/Minus | | |
| | Identities = 300/302 (99%). | emb AL591978.1 Listeria monocytogenes strain EGD, complete genome, segment 6/12 | |
| | Gaps = 0/302 (0%), | | |
| | Strand=Plus/Minus | | |
| Sequence 1 Clone 1/PL95 | Identifies = 165/168 (98%), | emb AJ4174492 Shuttle integration vector pPL2 | |
| | Gaps = 1/168 (0%), | | |
| | Strand=Plus/Minus | | |
| | Identities = 292/293 (99%), | emb AL591978.1 Listeria monocytogenes strain EGD, complete genome, segment 6/12 | |
| | Gaps = 0/293 (0%), | | |
| | Strand=Plus/Minus | | |
| Sequence 1 Clone 1/PL95 | Identities = 171/174 (98%). | emb AJ417449.2 Shuttle integration vector pPL2 | |
| | Gaps = 2/174 (1%), | | |
| | Strand=Plus/Minus | | |
| | Identities = 301/302 (99%), | emb AL591978.1 Listeria monocytogenes strain EGD, complete genome, segment 6/12 | |
| | Gaps = 0/302 (0%), | | |
| | Strand=Plus/Minus | | |
| Sequence 1 Clone 1/PL95 | Identities = 167/168 (99%), | emb AJ417449.2 Shuttle integration vector pPL2 | |
| | Gaps = 1/168 (0%), | | |
| | Strand=Plus/Minus | | |
| | Identities = 300/302 (99%), | emb AL591978.1 Listeria monocytogenes strain EGD, complete genome, segment 6/12 | |
| | Gaps = 0/302 (0%), | | |
| | Strand=Plus/Minus | | |

| | | | |
|---|---|---|---|
| *a* Refereed to by blast report *b* As obtained by sequencing | | | |

Restriction digestion is performed using FastDigest^{®} restriction enzymes BamHI and SalI available from Fermentas (Fermentas International Inc., Burlington, Canada) according to manufacturer's instructions. A total reaction volume of 20 µl is incubated for one hour at 37°C. Dephosphorylation of the vector and the IAC fragments before ligation is performed using FastAP™ Thermosensitive Alkaline Phosphatase (Fermentas) according to manufacturer's instructions for 30 min at 37°C. T4 DNA ligase (Fermentas) is used for ligation of the vector pPL2 and the artificial IAC fragment over night at 4°C.
Real-time PCR detection of *L. monocytogenes* by targeting a 274 bp fragment of the *prfA* gene is performed according to previously published format using the primers Lip1 and Lip2 and FAM-labelled Lip-probe (D'Agostino et al. (2004) J. Food Prot. 67, 1646-1655; Rossmanith et al. (2006) Res. Microbiol. 157, 763-771). Real-time PCR detection of the artificial IAC fragment is performed according to Rossmanith et al. (2006) using Lip1 and Lip2 and HEX-labelled pLucLm4.
The forward primer (Lip1:5'-GATACAGAAACATCGGTTGGC-3') and the reverse primer (Lip2:5'-GTGTAATCTTGATGCCATCAGG-3') amplify a 274 bp fragment of the *prfA* gene. Two different TaqMan™ probe formats with increased melting temperature are used. The Lip-probe (5'-FAM-CAGGATTAAAAGTTGACCGCA-MGB - 3') uses an MGB modification. The probe for the IAC of the assay (pLucLm 4: 5'-HEX-TTCGAAATGTCCGTTCGGTTGGC -BHQ1-3') is HEX labelled. Primers and probe pLucLm 4 can be purchased at MWG Biotech (Ebersberg, Germany). The MGB-modified probe is purchased at Applied Biosystems. Conventional PCR for confirmation of the insertion of pPL2-IAC into the Δ-*prfA L. monocytogenes* EGDe genome is performed using primers NC16 and PL95 (Lauer et al. (2002) J. Bacteriol. 184, 4177-4186). IAC+, Δ-*prfA L. monocytogenes* EGDe is identified as *L. monocytogenes* EGDe by amplifying the *iap*- locus of *L. monocytogenes* according to Bubert et al. (1999, Appl. Environ. Microbiol. 65, 4688-4692) and targeting the 16S rRNA gene specific for all *Listeria* spp. and the *hly* gene specific for *L*. *monocytogenes* according to Border et al. (1990, Lett. Appl. Microbiol. 11, 158-162).
Conventional and real-time PCR reactions are performed in an Mx3000p real-time PCR thermocycler (Stratagene, La Jolla, CA, USA). The 25 µl volume contains 20 mM Tris-HCl, 50 mM KCI, 3.5 mM MgCl₂, 500 nM of each primer, 250 nM of each probe, 200 µM (each) of dATP, dTTP, dGTP and dCTP, 1.5 U of Platinum^{©} *Taq* DNA polymerase (Invitrogen, Lofer, Austria) and 5 µl isolated DNA. Amplification following initial denaturation at 94 °C for 2 min is performed in 45 cycles, at 94 °C for 15 s, and 64 °C for 1 minute.
For the DNA standard for real-time PCR quantification one millilitre of a pure culture of *L. monocytogenes* strain EGDe is subjected to DNA isolation using the NucleoSpin^{©} tissue kit and the support protocol for Gram-positive bacteria. The DNA concentration is measured fluorimetrically using a Hoefer DyNA Quant200 device (Pharmacia Biotech). The copy number of the *prfA* gene is determined by assuming that, based on the molecular weight of the genome of *L*. *monocytogenes,* 1 ng of DNA equals 3.1 × 10⁵ copies of the entire genome, and that the *prfA* gene is a single-copy gene. The slope (s) of the standard curve is used for calculation of the PCR efficiency (*E*) with the following equation: *E* = 10^{-1/s} - 1 [21].

Real-time PCR results are expressed as bacterial cell equivalents (BCE). The copy number of the *prfA* gene and the IAC insert is determined by assuming that, based on the molecular weight of the genome of *L*. *monocytogenes,* 1 ng of DNA equalled 3.1 × 10⁵ copies of the entire genome, and that the *prfA* gene and the IAC insert are a single-copy gene and a single copy insert within the genome respectively (Nelson et al.(2004) Nucleic Acids Res. 32, 2386-2395). All real-time PCR reactions are performed in duplicate except as noted otherwise.
PCR products of conventional PCR are separated in 1.5 % agarose gels at 90 V for 25 min and stained with 0.5 µg/ml ethidium bromide (Sigma-Aldrich GmbH, Steinheim, Germany). GeneRuler 100 bp (MBI Fermentas, St. Leon-Rot, Germany) was used as a standard.

**1.3. DNA extraction and measurement.** The genomic DNA of one millilitre overnight bacterial culture is extracted by using the NucleoSpin^{®} tissue kit (Macherey - Nagel) and the support protocol for Gram-positive bacteria. Plasmid DNA for cloning experiments is extracted using the Quiagen Plasmid Midi Kit (Hilden, Germany) according to manufacturer's instructions. DNA concentration is analytically determined by fluorimetric measurement using a Hoefer DyNA Quant200 apparatus (Pharmacia Biotech, San Francisco, CA, USA) and a 8452A Diode Array Spectrophotometer (Hewlett Packard, Palo Alto, CA, USA).

**1.4. Cloning of pPL2-IAC.** Adequate amounts of the IAC (IAC: 5'-GAT ACA GAA ACA TCG GTT GGC GTA TTC GAA ATG TCC GTT CGG TTG GCG CTA TGA AGA GAT ACG CGG TGG AAC CTG GAA CCT GAT GGC ATC AAG ATT ACA C-3') for cloning are produced by amplification of the 100 bp fragment by conventional PCR according to Rossmanith et al. (2006, Res. Microbiol. 157, 763-771) using the modified primers LipBam and LipSal containing the restriction sites BamHI and SalI. After purification of the amplification product using NucleoSpin^{®} Extract II (Macherey-Nagel) restriction digestion and dephosphorylation are performed as described above, following ligation with the vector pPL2. Prior to ligation the phage integration vector pPL2 is transformed into *E*. *coli* TOP10F' by standard heat shock techniques (Sambrook et al. (1989) Molecular cloning: a laboratory manual, 2nd ed. Cold Spring Harbor Laboratory Press, Cold Spring Harbor, N.Y.) for amplification. The vector is extracted as described above and used for ligation after purification, restriction digestion and dephosphorylation. The resulting plasmid pPL2-IAC is then transformed into *E. coli* TOP10F' via heat-shock. pPL2-IAC positive clones are selected by plating on Luria-Bertani broth (LB; Oxoid) containing 25 µg/ml of chloramphenicol.

Single bacterial colonies are picked up and screened for positive transformation *of E. coli* with pPL2-IAC. Restriction digestion analysis results in two fragments of a length of ~ 6,000 bp and 100 bp corresponding to the lengths of vector and insert indicating transformation of pPL2-IAC into *E. coli.* Real-time PCR targeting the IAC using pLucLm4 results in amplification of the target, thus confirming the restriction digestion analysis (Fig. 1A).

**1.5. Transformation of pPL2-IAC *into* Δ-*prfA L. monocytogenes* EGDe..** Δ-*prfA L. monocytogenes* EGDe is transformed with pPL2-IAC using a modified electroporation protocol (Park et al. (1990) Gene 94, 129-132). Electro-competent Δ-*prfA L. monocytogenes* EGDe cells are prepared as follows: Cells are grown in brain heart infusion broth (BHI; Oxoid) containing 5 µg/ml penicillinG to an OD₆₀₀ of 0.3 at 37°C. Cells are harvested at 8000 × g, 10 min at 4°C and the pellet is washed twice in 1/10 vol. 3.5 × SMHEM buffer (72 mM sucrose, 1 mM MgCl₂, and 2 mM HEPES). After resuspending the pellet in 1/100 vol. 3.5 × SMHEM buffer 100 µl aliquots are stored at -80°C.
For electroporation 100 µl aliquots of electro-competent Δ-*prfA L. monocytogenes* EGDe are thawed on ice and mixed with 5 µl DNA suspension (~ 1 µg/reaction) containing pPL2-IAC. After incubation for one min on ice, electroporation is performed (100 Ω, 50 µF, 1000 V) in a Gene Pulser Xcell^{™} electroporation system (Bio-Rad Laboratories, Inc, Hercules, CA, USA). One millilitre of pre warmed BHI is added, the sample is incubated 6 h at 37°C and subsequently plated on BHI agar containing 25 µg/ml of chloramphenicol. After 24 - 48 h incubation at 37°C individual colonies are picked and screened for pPL2-IAC positive Δ-*prfA L. monocytogenes* EGDe clones.

**1.6. Confirmation of IAC+, Δ-*prfA L. monocytogenes* EGDe.** Four chloramphenicol screening positive clones of IAC+, Δ-*prfA L. monocytogenes* EGDe are confirmed by testing the presence of the IAC sequence in Δ-*prfA L. monocytogenes* EGDe. This is performed by amplifying the 100 bp fragment of the IAC using the probe pLucLm4. Genomic DNA from IAC+, Δ*-prfA L. monocytogenes* EGDe is subjected to real-time PCR resulting in positive amplification (Fig. 1 B). The tested clones achieve a mean Ct-value of 11.5 (SD (standard deviation): 0.16) corresponding to a mean of 5.36 × 10⁷ copies. The Δ*-prfA* status of the cloned strain is tested by real-time PCR using the probe Lip-probe hybridizing the 274 bp fragment of the *prfA* locus of the wild type *L*. *monocytogenes* resulting in no amplification.
Integration of the vector within the genome is tested with conventional PCR as described under paragraph 1.2. The resulting fragments after electrophoresis correspond to the proposed length of the amplification product of 499 bp (Fig 2C). This indicates insertion of the vector into the *Listeria* genome as the primers NC16 and PL95 allow amplification across the attachment site tRNA^{Arg}-*attBP'* in *L*. *monocytogenes* serotype 1/2a resulting in a hybrid amplificate containing parts of the sequences of the vector (3') and of the *Listeria* genome (5').
Sequencing analysis of IAC+, Δ-*prfA L. monocytogenes* EGDe is performed using this hybrid PCR amplificate using primers NC16 and PL95. The obtained sequence is 100% identical with a 210 bp fragment of the shuttle integration vector pPL2 and 100% identical to a 261 bp fragment of *L*. *monocytogenes* including the proposed attachment site tRNA^{Arg}-*attBP'* (Strain EGDe, complete genome, segment 6/12; mb/AL591978.1). Confirmation testing of the resulting IAC+, Δ-*prfA* strain to be *L*. *monocytogenes* EGDe is additionally performed by conventional PCR as described under paragraph 1.2. The results are presented in Fig 2A and B. demonstrating matching fragment sizes for IAC+, Δ-*prfA L. monocytogenes* EGDe and the control *L. monocytogenes* EGDe for both PCR assays.

**1.6. IAC+, Δ-*prfA L. monocytogenes* EGDe sequencing.** PCR products from positive clones after amplification are purified using the NucleoSpin^{®} Extract II Kit (Macherey-Nagel), following the manufacturer's instructions. Purified PCR products are submitted to Macrogen Inc. (Seoul, Korea) for DNA sequencing. Samples are sequenced on both strands using NC16 and PL95 primers on the cycle sequencing reaction.
The obtained sequences are analyzed and aligned using the BLAST server available at the National Center for Biotechnology Information website (hftp://www.ncbi.nlm.nih.gov/BLAST/).

**1.7. Quantitative real-time PCR for confirmation of single copy insertion of pPL2-IAC into the genome of Δ-*prfA L. monocytogenes* EGDe and calibration against *L. monocytogenes* EGDe wild type data.** Calibration standards of genomic IAC+, Δ-*prfA L. monocytogenes* EGDe DNA and *L. monocytogenes* EGDe wild type are compared using the *prfA* real-time PCR assay in a multiplex system with the probes pLucLm4 and Lip-probe. Based on the assumption that *prfA* is a single copy gene ten fold dilutions of both DNA targets are compared. The ten-fold dilutions are starting at 1.58 × 10⁶ copies equalling 5 ng of genomic DNA as determined by fluorimetric and UV-VIS measurement. The results are presented in Table 3 and show good concordance of the Ct-values of both standard series. This also demonstrates comparable performance of both probes within the reactions. The underlying data result from duplicates repeated in four real-time PCR runs. For confirmation of these results the concentrations of cultures of IAC+, Δ-*prfA L. monocytogenes* EGDe are determined by viability staining and compared to real-time PCR results. The number of 3.2 × 10³ (RSD.: 6.7%) BCE per sample as obtained by real-time PCR compares to 2.8 × 10³ (RSD.: 25.0%) CFU per sample as determined by viable staining.

**Table 3.**

| Comparsion of Ct-values after real-time PCR derived from a ten-fold standard dilution of genomic DNA of *L. monocytogenes* EGDe wild type and the cloned EGDe Δ-*prf,* IAC+ strain demonstrating single copy insertion of pPL2-IACa into the genome of *L. monocytogenes* EGDe Δ-*prfA*. | | | |
|---|---|---|---|
| | Strain | | |
| | *EGDe* Δ-*prfA,* IAC+ | EGDe wt | EGDe wt against EGDe Δ-prfA, IAC+ background |
| | Monoplex PCR | | Duplex PCR |
| Target Copy Nr.*^{b}* | Ct-values (SD)*^{c}* | Ct-values (SD)*^{d}* | Ct-values (SD)*^{d,e}* |
| 1.58 × 10⁶ | 16.8 (0.08) | 16.6 (0.11) | - |
| 1.58 × 10⁵ | 20.1 (0.13) | 20.0 (0.02) | 19.9 (0.13) |
| 1.58 × 10⁴ | 23.4 (0.14) | 23.1 (0.18) | 23.4 (0.26) |
| 1.58 × 10³ | 26.8 (0.13) | 26.7 (0.06) | 26.7 (0.31) |
| 1.58 × 10² | 30.4 (0.07) | 30.1 (0.02) | 29.9 (0.68) |
| 1.58 × 10¹ | 33.8 (0.27) | 34.1 (0.91) | 33.4 (0.77) |

| | | | |
|---|---|---|---|
| *a* Based on the assumption that *prfA* is a single copy gene. *b* Dilution series derived from a solution containing 1ng/µl genomic DNA representing 1.58 × 10⁶ copies in 5µl DNA template. *c prfA* real-time PCR assay using HEX labelled pLucLM4 probe, amplifying the artificial IAC sequence. *d prfA* real-time PCR assay using FAM labelled Lip-probe, amplifiying 274 bp of the *prfA* locus sequence. *e* Genomic DNA of *L. monocytogenes EGDe* against 1.58 × 10⁵ copies background of the genome of the EGDe Δ-*prfA,* IAC+ strain in a multiplex reaction. | | | |

The influence of increasing amounts of genomic DNA derived from IAC+, Δ-*prfA L. monocytogenes* EGDe on the performance of the main reaction amplifying the *prfA* locus of the wild type strain and main bacterial target is investigated with multiplex real-time PCR. Using Lip-probe and pLucLm4, ten-fold dilutions of genomic *L. monocytogenes* EGDe wild type DNA starting at 1.58 × 10⁶ copies to 1.58 × 10¹ are tested in an ascending as well as descending matrix against a background of ten-fold dilutions of 1.58 × 10⁶ to 1.58 × 10¹ copies of genomic IAC+, Δ-*prfA L. monocytogenes* EGDe DNA. The resulting Ct values for all tested combinations deviate from the respective values as obtained by the monoplex experiments of the genomic standards of both strains with a mean standard deviation of 0.3 for all combinations of standard concentration. Exemplarily the resulting Ct-values for a tenfold dilution of genomic DNA of *L. monocytogenes* EGDe are presented in Table 3 against a background of 1.58 × 10⁵ copies of IAC+, Δ-*prfA L. monocytogenes* EGDe.

**1.8. Artificially and naturally contaminated food samples.** UHT milk for artificial contamination is purchased at local supermarkets and tested to be *L. monocytogenes* negative prior to inoculation performing real-time PCR targeting the *prfA* locus of *L*. *monocytogenes.* Artificial contamination is performed using a 10-fold dilution series in Ringer's solution (Oxoid) of a pure culture of *L. monocytogenes* EGDe containing 8.5 × 10⁸ CFU/ml. 100 µl of the appropriate dilutions are added to the samples. The dilution series is prepared to contain 10¹-10², 10²-10³, 10³-10⁴ and 10⁴-10⁵ CFU/ml. The number of CFU for each step of the dilution series is obtained by the plate count method using tryptone soy agar with 0.6% yeast (TSA-Y; Oxoid). The experiment is performed in triplicates.
Naturally contaminated soft cheese samples are provided by regulatory authority and originated from a recent *L*. *monocytogenes* outbreak in Styria, Austria and are stored at 4°C. Two samples from two different production charges are processed in quadruplicates resulting in eight individual samples. Additionally, ISO 11290-2 is carried out accordingly for each sample to compare the quantitative results of the experiments with this standard method (ISO 11290-2; ISO 11290-2/Amd1).

**1.9. Sample Treatment.** Sample treatment is performed using the matrix lysis protocol as published by Mester et al. (2010) J. Food Protect. 73, 680-687 and Rossmanith et al. (2007) J. Microbiol. Methods 69, 504-511.

### 1.10. Statistical analysis.

Two-group comparisons are analyzed by chi square test. P values are calculated, and values ≤0.05 are considered significant.

### 2. Application Examples:

**2.1. Phenotype of** IAC+, **Δ-*prfA L. monocytogenes*** EGDe **on OCLA, PALCAM, blood agar and RAPID' L.mono agar media.** On blood agar IAC+, Δ-*prfA L. monocytogenes* EGDe show no haemolysis. On PALCAM agar IAC+, Δ-*prfA L. monocytogenes* EGDe show the same phenotype as the *L. monocytogenes* EGDe wild type, as presented in Table 2. The morphology and colour of IAC+, Δ-*prfA L. monocytogenes* EGDe on OCLA is also similar to the wild type except the halo formation which is not observable for IAC+, Δ-*prfA L. monocytogenes* EGDe after 24 and 48 h of incubation. After 72 h of incubation IAC+, Δ-*prfA L. monocytogenes* EGDe show some weak halo formation as well. Plated on RAPID' L.mono the cloned strain develops white colour, lacking the characteristic green colour of the *L. monocytogenes* EGDe wild type strain on RAPID' L.mono agar.

**TABLE 2.**

| Colony morphology of the cloned *L*. *monocytogenes* EGDe Δ*prfA,* IAC+ strain in comparsion to the wild type *L*. *monocytogenes* EGDe and *L. innocua* on selected selective and chromogenic agar media. | | | | | |
|---|---|---|---|---|---|
| Strain/Media | | RAPID' L.mono | OCLA | PALCAM | Blood agar |
| *L. monocytogenes* EGDe Wildtype | Morphology | - | Halo formation*^{a}* | Fish eye | - |
| | Colour | Green | Blue | Green | - |
| | Agar staining | no | - | Black | Haemolysis |
| *L. monocytogenes* EGDe ΔprfA, IAC+ | Morphology | - | Halo after 72 h*^{b}* | Fish eye | - |
| | Colour | White | Blue | Green | - |
| | Agar staining | no | - | Black | No haem. |
| *L. innocua* | Morphology | - | No halo | Fish eye | - |
| | Colour | White | Blue | Green | - |
| | Agar staining | Yellow | - | Black | No haem. |

| | | | | | |
|---|---|---|---|---|---|
| a Halo formation was finished after 24 h incubation at 37°C for *L. monocytogenes EGDe wildtype.* *b* No halo was observed for *L*. *monocytogenes* EGDe Δlpha, IAC+ after 24 h and 48 h of incubation. | | | | | |

**2.2. Application of IAC+, Δ-*prfA L. monocytogenes* EGDe as internal process control to artificial contaminated UHT milk and naturally contaminated Quargel cheese.** Artificially as well as naturally contaminated food samples are processed according to the method disclosed by Rossmanith et al. (2007) J. Microbiol. Methods 69, 504-511: This method is based on sample preparation by lysis of the food matrix and subsequent separation of the target bacteria using centrifugation followed by DNA isolation and real-time PCR detection. The ionic liquid 1-ethyl-3-methylimidazolium thiocyanate ([emim]SCN) is used as solvent during sample preparation.

For artificial contamination 12.5 g samples of UHT milk are spiked with a four-step decimal dilution series of *L. monocytogenes* EGDe wild type as described in section 1.8. Additionally, 1.4 × 10³ (RSD.: 29.0%) CFU per sample of the internal sample process control IAC+, Δ-*prfA L. monocytogenes* EGDe are added.

The main target pathogen *L. monocytogenes* EGDe wild type is recovered from UHT milk by a factor of 55% (RSD (relative standard deviation): 10.9%) compared with microscopy count. The relative standard deviation within the log-scales is 10.9% representing a high level of precision and reproducibility in terms of log scale recovery and indicating no biasing influence of the internal sample process control by means of IAC+, Δ-*prfA L. monocytogenes* EGDe cells.
The internal sample process control indicates a recovery of 49% (RSD.: 27.1%) compared with microscopic count. Initially 1.4 × 10³ (RSD.: 29.0%) CFU (colony forming unit) per sample results in 6.9 × 10² (RSD.: 27.0%) BCE after recovery and real-time PCR detection.
The system is then applied to naturally contaminated samples of Quargel cheese. Additionally 1.4 × 10³ (RSD.: 29.0%) CFU IAC+, Δ-*prfA L. monocytogenes* EGDe are added per sample.
The samples are processed in two ways: A direct quantification of the value of *L. monocytogenes* is obtained by matrix lysis and subsequent real-time PCR. Also ISO 11290-2 is performed for comparison. Additionally the samples are processed by matrix lysis and subsequent real-time PCR using a DNA isolation protocol omitting the Proteinase K step. This artificially biases the digestion performance and resulting thereof the efficiency of the whole protocol.
The values of *L. monocytogenes* contamination of the samples directly obtained after real-time PCR reach an average of 1.4 × 10⁶ (RSD.: 5.9%) BCE and 1.1 × 10⁸ (RSD.: 8.9%) BCE for the two respective Quargel cheese charges. These values are corrected according to the rate of efficiency for each sample as obtained by comparing the values of each replicate to the average value of the IAC+, Δ-*prfA L. monocytogenes* EGDe process control of 6.9 × 10² (RSD.: 5.9%) BCE after real-time PCR. After this correction the *L. monocytogenes* contamination average 1.1 × 10⁶ (RSD.: 35.8%) BCE and 2.3 × 10⁸ (RSD.: 10.2%) for the two charges. A further correction including the overall loss of IAC+, Δ-*prfA L. monocytogenes* EGDe process control cells from initially 1.4 × 10³ (RSD.: 29.0%) CFU to 6.9 × 10² (RSD.: 5.9%) BCE after real-time PCR is done. This results in 2.32 × 10⁶ (RSD.: 36.0%) BCE/g and 5.0 × 10⁸ (RSD.: 9.9%) BCE/g for the two cheese charges comparing to 2.07 × 10⁶ (RSD.: 91.1 %) CFU/g and 4.9 × 10⁸ (RSD.: 73.2%) CFU/g as obtained by ISO 11290-2. The samples processed by artificially biased DNA isolation average in basic values of 4.9 × 10⁵ (RSD.: 6.3%) BCE and 3.7 × 10⁷ (RSD.: 2.9%) for the respective cheese charges. After correction as prescribed above the respective *L. monocytogenes* contamination is 1.8 × 10⁶ (RSD.: 47%) BCE and 4.6 × 10⁸ (RSD.: 10.1%) BCE.

## Claims

1. A genetically modified bacterium of the species *Listeria monocytogenes,* wherein the genomic locus of the transcriptional factor PrfA has been deleted, **characterised in that** it comprises on genomic level an artificial sequence that acts as internal amplification control (IAC).

2. The *Listeria monocytogenes* bacterium of claim 1, wherein the artificial sequence that acts as IAC comprises primer binding sequences at the 5' and 3' ends.

3. The *Listeria monocytogenes* bacterium of claim 2, wherein the primer binding sequences are identical to the primer binding sequences of the genomic *prfA* locus of wild type *Listeria monocytogenes.*

4. A *Listeria monocytogenes* bacterium of any of the claims 1 - 3, wherein the artificial sequence that acts as IAC is a 100 bp sequence according to SEQ ID NO: 1.

5. A genetically modified *Listeria monocytogenes* EGDe strain of any of the claims 1-4.

6. A genetically modified *Listeria monocytogenes* EGDe strain according to one or more of claims 1 to 5, wherein the genomic locus of the transcriptional factor PrfA has been deleted, comprising the internal amplification control sequence (IAC) of SEQ ID NO:1 deposited at the DSMZ as *Listeria monocytogenes* AΔrfA/+IAC under DSM 23639 on Mai 20, 2010.

7. Use of genetically modified *Listeria monocytogenes* as specified in any of the claims 1 - 6 for detecting and determining qualitatively and / or quantitatively the occurrence of wild type *Listeria monocytogenes* in a sample suspected to be contaminated with said micro-organism.

8. Use according to claim 7, **characterized in that** the sample is food.

9. Use of genetically modified *Listeria monocytogenes* as specified in any of the claims 1 - 6 as internal sample process control (ISPC) for a real-time PCR based assay.

10. A method for detecting and determining the occurrence of wild type *Listeria monocytogenes* in a sample suspected to be contaminated with said pathogenic micro-organism, comprising the steps:
(a) adding to said sample a predefined amount of cells of the genetically modified *Listeria monocytogenes* bacterium as specified in any of the claims 1 - 6,
(b) incubating the sample with an extraction solution,
(c) isolating the DNA by standard methods;
(d) applying real-time PCR, thereby using (i) primers specific for the genomic *prfA* locus of wild type *Listeria monocytogenes* and the IAC sequence of genetically modified *Listeria monocytogenes* as specified in any of the claims 1 - 6; and (ii) a fluorescent labelled oligonucleotide probe that is able to specifically hybridize with said *prfA* locus and a fluorescent labelled oligonucleotide probe that is able to specifically hybridize with said IAC sequence,
(e) determining qualitatively and / or quantitatively fluorescent signals generated by step (d), and
(f) determining and / or calculating from step (e) the presence and / or the amount of the wild type *Listeria monocytogenes* cells in the original sample suspected to be contaminated with said micro-organism.

11. The method according to claim 10, wherein the primers used in step (d)(i) are Lip1 (SEQ ID NO: 2) and Lip2 (SEQ ID NO: 3).

12. The method of claim 10 or 11, wherein the fluorescent labelled probes used in step (d)(ii) are pLucLm4 (SEQ ID NO:4) for detecting the IAC sequence, and LipProbe (SEQ ID NO:5) for detecting the *prfA* locus.

13. A method according to any of the claims 10-12, wherein the extraction solution of step (b) comprises at least MgCl₂ and/or an ionic liquid.

14. A kit for use in an assay for detecting and determining wild type *Listeria monocytogenes* in a sample suspected to be contaminated with said micro-organism, comprising at least in one or more packages
(i) genetically modified *Listeria monocytogenes* as specified in any of the claims 1 - 6;
(ii) the primers specific for the genomic *prfA* locus of wild type *Listeria monocytogenes* and the IAC sequence of genetically modified *Listeria monocytogenes* as specified in any of the claims 1 - 6; and
(iii) a fluorescent labelled oligonucleotide probe that is able to specifically hybridize with said *prfA* locus and a fluorescent labelled oligonucleotide probe that is able to specifically hybridize with said IAC sequence.

15. The kit according to claim 14, wherein the primers are Lip1 (SEQ ID NO:2) and Lip2 (SEQ ID NO:3).

16. The kit according to claim 14 or 15, wherein the fluorescent labelled probes are pLucLm4 (SEQ ID NO:4) for detecting the IAC sequence and LipProbe (SEQ ID NO:5) for detecting the *prfA* locus.

17. A method for manufacturing a genetically modified *Listeria monocytogenes* bacterium as specified in any of the claims 1 - 6 comprising the steps:
(a) deleting the genomic locus of the transcriptional factor PrfA of the bacterial species *Listeria monocytogenes,*
(b) cloning of a vector comprising an IAC,
(c) transforming the vector of step (b) into the bacterial species of step (a).

## Patentansprüche

1. Genetisch modifiziertes Bakterium der Spezies *Listeria monocytogenes,* worin der genomische Locus des Transkriptionsfaktors PrfA deletiert ist, **dadurch gekennzeichnet, dass** es auf genomischer Ebene eine künstliche Sequenz umfasst, die als interne Amplifikationskontrolle (IAC) dient.

2. *Listeria monocytogenes-*Bakterium nach Anspruch 1, wobei die künstliche Sequenz, die.als IAC dient, Primerbindungssequenzen an den 5'-und 3'-Enden umfasst.

3. *Listeria monocytogenes-Bakterium* nach Anspruch 2, wobei die Primer-bindungssequenzen identisch mit den Primerbindungssequenzen des genomische *prfA*-Locus von Wildtyp-*Listeria monocytogenes* sind.

4. *Listeria monocytogenes-Bakterium* nach einem der Ansprüche 1 - 3, wobei die künstliche Sequenz, die als IAC dient, eine 100 bp lange Sequenz gemäß SEQ ID NO: 1 ist.

5. Genetisch modifizierter *Listeria monocytogenes*-EGDe-Stamm nach einem der Ansprüche 1 - 4.

6. Genetisch modifizierter *Listeria monocytogenes*-EGDe-Stamm nach einem oder mehreren der Ansprüche 1 bis 5, worin der genomische Locus des Transkriptionsfaktors PrfA deletiert ist, umfassend die interne Amplifikationskontrollsequenz (IAC) der SEQ ID NO: 1, hinterlegt bei der DSMZ als *Listeria monocytogenes* ΔprfA/+IAC unter DSM 23639 am 20. Mai 2010.

7. Verwendung von genetisch modifiziertem *Listeria monocytogenes* nach einem der Ansprüche 1 - -6 zum Nachweis und zur qualitativen und/oder quantitativen Bestimmung des Vorkommens von Wildtyp-*Listeria monocytogenes* in einer Probe, von der angenommen wird, dass sie mit dem Mikroorganismus kontaminiert ist.

8. Verwendung nach Anspruch 7, **dadurch gekennzeichnet, dass** es sich bei der Probe um Lebensmittel handelt.

9. Verwendung von genetisch modifiziertem *Listeria monocytogenes* nach einem der Ansprüche 1 - 6 als interne Probenaufbereitungskontrolle (internal sample process control, ISPC) für einen Assay auf der Basis von Echtzeit-PCR.

10. Verfahren zum Nachweis und zur Bestimmung des Vorkommens von Wildtyp-*Listeria monocytogenes* in einer Probe, von der angenommen wird, dass sie mit dem pathogenen Mikroorganismus kontaminiert ist, umfassend die Schritte:
(a) Zugeben zu der Probe einer festgelegten Menge an Zellen des genetisch modifizierten *Listeria monocytogenes*-Bakteriums nach einem der Ansprüche 1 - 6,
(b) Inkubieren der Probe mit einer Extraktionslösung,
(c) Isolieren der DNA gemäß Standardverfahren,
(d) Anwenden von Echtzeit-PCR, wobei man Folgendes verwendet: (i) Primer, die spezifisch für den genomischen *prfA*-Locus von Wildtyp-*Listeria monocytogenes* und die IAC-Sequenz von genetisch modifiziertem *Listeria monocytogenes* nach einem der Ansprüche 1 - 6 sind, und (ii) eine fluoreszenzmarkierte Oligonukleotidsonde, die spezifisch mit dem *prfA*-Locus hybridisieren kann, und eine fluoreszenzmarkierte Oligonukleotidsonde, die spezifisch mit der IAC-Sequenz hybridisieren kann,
(e) qualitatives und/oder quantitatives Bestimmen von Fluoreszenzsignalen, die durch Schritt (d) hervorgerufen werden, und
(f) Bestimmen und/oder Berechnen aus Schritt (e) des Vorhandenseins und/oder der Menge der Wildtyp-*Listeria monocytogenes*-Zellen in der ursprünglichen Probe, von der angenommen wird, dass sie mit dem Mikroorganismus kontaminiert ist.

11. Verfahren nach Anspruch 10, wobei es sich bei den in Schritt (d)(i) verwendeten Primern um Lip1 (SEQ ID NO: 2) und Lip2 (SEQ ID NO: 3) handelt.

12. Verfahren nach Anspruch 10 oder 11, wobei es sich bei den in Schritt (d)(ii) verwendeten fluoreszenzmarkierten Sonden um pLucLm4 (SEQ ID NO: 4) zum Nachweis der IAC-Sequenz und LipProbe (SEQ ID NO: 5) zum Nachweis des *prfA*-Locus handelt.

13. Verfahren nach einem der Ansprüche 10 - 12, wobei die Extraktionslösung von Schritt (b) zumindest MgCl₂ oder eine ionische Flüssigkeit umfasst.

14. Kit für die Verwendung in einem Assay zum Nachweis und zur Bestimmung von Wildtyp-*Listeria monocytogenes* in einer Probe, von der angenommen wird, dass sie mit dem Mikroorganismus kontaminiert ist, das in einer oder mehreren Packungen mindestens Folgendes umfasst:
(i) genetisch modifiziertes *Listeria monocytogenes* nach einem der Ansprüche 1 - 6,
(ii) die Primer, die spezifisch für den genomischen *prfA*-Locus von Wildtyp*-Listeria monocytogenes* und die IAC-Sequenz von genetisch modifiziertem *Listeria monocytogenes* nach einem der Ansprüche 1 - 6 sind, und
(iii) eine fluoreszenzmarkierte Oligonukleotidsonde, die spezifisch mit dem *prfA*-Locus hybridisieren kann, und eine fluoreszenzmarkierte Oligonukleotidsonde, die spezifisch mit der IAC-Sequenz hybridisieren kann.

15. Kit nach Anspruch 14, wobei es sich bei den Primern um Lip1 (SEQ ID NO: 2) und Lip2 (SEQ ID NO: 3) handelt.

16. Kit nach Anspruch 14 oder 15, wobei es sich bei den fluoreszenzmarkierten Sonden um pLucLm4 (SEQ ID NO: 4) zum Nachweis der IAC-Sequenz und LipProbe (SEQ ID NO: 5) zum Nachweis des *prfA-*Locus handelt.

17. Verfahren zur Herstellung von genetisch modifiziertem *Listeria monocytogenes*-Bakterium nach einem der Ansprüche 1 - 6, umfassend die Schritte:
(a) Deletieren des genomischen Locus des Transkriptionsfaktors PrfA der Bakterienspezies *Listeria monocytogenes,*
(b) Klonieren eines Vektors, der eine IAC umfasst,
(c) Transformieren des Vektors von Schritt (b) in die Bakterienspezies von Schritt (a).

## Revendications

1. Bactérie génétiquement modifiée de l'espèce *Listeria monocytogenes,* dans laquelle le locus génomique du facteur de transcription PrfA a été supprimé, **caractérisée en ce qu'**elle comprend au niveau génomique une séquence artificielle qui opère en tant que contrôle interne d'amplification (IAC).

2. Bactérie *Listeria monocytogenes* selon la revendication 1, dans laquelle la séquence artificielle qui opère en tant que IAC comprend des séquences de liaison d'amorçage au niveau des extrémités 5' et 3'.

3. Bactérie *Listeria monocytogenes* selon la revendication 2, dans laquelle les séquences de liaison d'amorçage sont identiques aux séquences de liaison d'amorçage de locus génomique prfA de la *Listeria monocytogenes* de type sauvage.

4. Bactérie *Listeria monocytogenes* selon l'une quelconque des revendications 1 - 3, dans laquelle la séquence artificielle qui opère en tant que IAC est une séquence 100 bp conformément à SEQ ID NO: 1.

5. Souche de *Listeria monocytogenes* EGDe génétiquement modifiée selon l'une quelconque des revendications 1 - 4.

6. Souche de *Listeria monocytogenes* EGDe génétiquement modifiée selon une ou plusieurs des revendications 1 à 5, dans laquelle le locus génomique du facteur de transcription PrfA a été supprimé, comprenant la séquence de contrôle interne d'amplification (IAC) de SEQ ID NO: 1 déposée à DSMZ en tant que *Listeria monocytogenes* ΔprfA/+IAC sous DSM 23639 le 20 Mai 2010.

7. Utilisation de *Listeria monocytogenes* génétiquement modifiée comme spécifié selon l'une quelconque des revendications 1 - 6 pour détecter et déterminer qualitativement et/ou quantitativement la présence de *Listeria monocytogenes* de type sauvage dans un échantillon suspecté d'être contaminé par ledit micro-organisme.

8. Utilisation selon la revendication 7, **caractérisée en ce que** l'échantillon est un aliment.

9. Utilisation de *Listeria monocytogenes* génétiquement modifiée comme spécifié selon l'une quelconque des revendications 1 - 6 en tant que contrôle interne de process d'échantillon (ISPC) pour un test basé sur PCR en temps réel.

10. Procédé pour détecter et déterminer la présence de *Listeria monocytogenes* de type sauvage dans un échantillon suspecté d'être contaminé par ledit micro-organisme pathogène, comprenant les étapes suivantes :
(a) ajout audit échantillon d'une quantité prédéfinie de cellules de la bactérie *Listeria monocytogenes* génétiquement modifiée comme spécifié selon l'une quelconque des revendications 1 - 6,
(b) incubation de l'échantillon avec une solution d'extraction,
(c) isolement de l'ADN au moyen de procédés standards,
(d) application de la PCR en temps réel, d'où ainsi l'utilisation (i) d'amorceurs spécifiques pour le locus prfA génomique de *Listeria monocytogenes* de type sauvage et de la séquence IAC de *Listeria monocytogenes* génétiquement modifiée comme spécifié selon l'une quelconque des revendications 1 - 6, et (ii) d'une sonde oligonucléotidique étiquetée par fluorescence qui peut s'hybridiser de façon spécifique avec ledit locus prfA et d'une sonde oligonucléotidique étiquetée par fluorescence qui peut s'hybridiser de façon spécifique avec ladite séquence IAC,
(e) détermination qualitative et/ou quantitative de signaux fluorescents générés au niveau de l'étape (d), et
(f) détermination et/ou calcul, à partir de l'étape (e), de la présence et/ou de la quantité des cellules de *Listeria monocytogenes* de type sauvage dans l'échantillon original suspecté d'être contaminé par ledit micro-organisme.

11. Procédé selon la revendication 10, dans lequel les amorceurs utilisés au niveau de l'étape (d)(i) sont Lip1 (SEQ ID NO: 2) et Lip2 (SEQ ID NO: 3).

12. Procédé selon la revendication 10 ou 11, dans lequel les sondes étiquetées par fluorescence utilisées au niveau de l'étape (d)(ii) sont pLucLm4 (SEQ ID NO: 4) pour détecter la séquence IAC et LipProbe (SEQ ID NO: 5) pour détecter le locus prfA.

13. Procédé selon l'une quelconque des revendications 10 - 12, dans lequel la solution d'extraction de l'étape (b) comprend au moins MgCl₂ et/ou un liquide ionique.

14. Kit pour une utilisation lors d'un test pour détecter et déterminer la *Listeria monocytogenes* de type sauvage dans un échantillon suspecté d'être contaminé par ledit micro-organisme, comprenant au moins un ou plusieurs modules constitué(s) par :
(i) la *Listeria monocytogenes* génétiquement modifiée comme spécifié selon l'une quelconque des revendications 1 - 6 ;
(ii) les amorceurs spécifiques pour le locus prfA génomique de la *Listeria monocytogenes* de type sauvage et la séquence IAC de la *Listeria monocytogenes* génétiquement modifiée comme spécifié selon l'une quelconque des revendications 1 - 6 ; et
(iii) une sonde oligonucléotidique étiquetée par fluorescence qui peut s'hybridiser de façon spécifique avec ledit locus prfA et une sonde oligonucléotidique étiquetée par fluorescence qui peut s'hybridiser de façon spécifique avec ladite séquence IAC.

15. Kit selon la revendication 14, dans lequel les amorceurs sont Lip1 (SEQ ID NO: 2) et Lip2 (SEQ ID NO: 3).

16. Kit selon la revendication 14 ou 15, dans lequel les sondes étiquetées par fluorescence sont pLucLm4 (SEQ ID NO: 4) pour détecter la séquence IAC et LipProbe (SEQ ID NO: 5) pour détecter le locus prfA.

17. Procédé pour fabriquer une bactérie *Listeria monocytogenes* génétiquement modifiée comme spécifié selon l'une quelconque des revendications 1 - 6, comprenant les étapes suivantes :
(a) suppression du locus génomique du facteur de transcription PrfA de l'espèce bactérienne *Listeria monocytogenes,*
(b) clonage d'un vecteur comprenant un IAC, et
(c) transformation du vecteur de l'étape (b) selon l'espèce bactérienne de l'étape (a).
